Europäisches Patentamt

European Patent Office

Office européen des brevets

(11) Publication number: **0 443 244 A1**

# EUROPEAN PATENT APPLICATION

(21) Application number: **90312451.9**

(51) Int. Cl.⁵: **A61F 13/15**

(22) Date of filing: **15.11.90**

(30) Priority: **22.02.90 US 484104**

(43) Date of publication of application:
**28.08.91 Bulletin 91/35**

(84) Designated Contracting States:
**AT BE DE ES FR GB GR IT SE**

(71) Applicant: **Paper Converting Machine Company**
**P.O. Box 19005**
**Green Bay WI 54307-9005(US)**

(72) Inventor: **Hertel, James E.**
**2777 N. Nicolet Drive**
**Green Bay, WI 54301(US)**
Inventor: **Merkatoris, John R.**
**308 Little Road**
**Green Bay, WI 54301(US)**

(74) Representative: **McCall, John Douglas et al**
**W.P. THOMPSON & CO. Coopers Building**
**Church Street**
**Liverpool L1 3AB(GB)**

(54) Method and apparatus for applying an elastic waistband to a disposable diaper.

(57) The invention includes a method and apparatus for applying an elastic waistband to disposable diapers. It uses canted, pin equipped wheels (15, 16) for stretching an elastic material and a belt system (24) for stripping the elastic material (14) from the pins (20) and applying it to a diaper component web (23).

FIG. 1

FIG. 3

This invention relates to a method and apparatus for applying an elastic waistband to a disposable diaper and, more particularly, to a unique method and apparatus for stretching the waistband in manufacture and developing the necessary gathering in the final product.

Disposable diapers typically include a moisture-pervious layer (non-woven) for positioning adjacent the body of an infant, an outer layer of moisture-impervious material, e.g. polyethylene, and an absorbent batt disposed between these two layers. These diapers also include elastic leg and waistbands fixedly secured to the layers of the diaper. The elastic bands gather the material around the waist and leg openings of the diaper and allow a user to secure the diaper snugly on an infant.

The prior art includes a wide variety of machines and methods used to produce disposable diapers. Some of these prior machines secure the elastic bands to the diaper and then heat shrink the band to develop the necessary gathering at the waist or leg opening. To heat the elastic, these prior machines use complicated mechanisms which reduce the rate of production of the machine. Other prior machines stretch the elastic band prior to securing it to the material layers of the diaper. However, these machines use complicated gripper mechanisms to stretch the elastic material and adhere or otherwise secure it to the layers or webs of the diaper.

Our Copending Application 89301697.2 (Publication No. 0338662) discloses apparatus for making disposable diapers having an elastic waistband, comprising a frame providing a longitudinally extending path, means on said frame for advancing a diaper component web in said path, means on said frame for advancing an elastic sheet material web toward said diaper component web, means operably associated with said frame for transversely cutting said web to provide transversely elongated segments therefrom, and means on said frame for applying stretched segments of said material to said diaper component web, characterised by a pair of spaced-apart substantially identical wheels mounted on said frame in said path for rotation about angularly related axes whereby the corresponding points on the peripheries of said wheels are closer together at a first position than at a second position spaced circumferentially from said first position, each wheel being equipped with generally radially outwardly extending pins on the periphery thereof, means on said frame for installing sheet material on said pins at said first position to span the space between said wheels and pressure roll means on said frame between said wheels for sequentially removing said segments from said pins at said second position and substantially simultaneously therewith adhesively uniting said material to said diaper component web.

The apparatus of the present invention provides an improved construction for removing the elastic sheet material from the pins and transferring it to the diaper component web.

In accordance with one embodimet of this invention, a pair of canted wheels equipped with generally radially extending pins on their peripheries receive a sheet of elastic material at a first position. As the wheels rotate, a pin on one wheel impales one end of the sheet, and a corresponding pin on the other wheel impales the opposite end of the sheet. After receiving the elastic material, the wheels continue to rotate, automatically stretching the elastic material as the pins move farther apart due to the cant of the wheels.

The wheels move the elastic sheet to a second position where they deposit it onto a web used to construct diapers. They deposit the elastic material on a portion of the web which receives adhesive before receiving the elastic sheet. A supporting roll supports the web while the web receives the elastic material. This supporting roll co-acts with a belt assembly to apply pressure to the stretched elastic sheet and insure that the adhesive secures it to the web.

The belt assembly includes two pairs of endless belts which are entrained on the wheels under the elastic material, then over the elastic material as it travels with and on the supporting roll, and finally around idler sheaves, thereby following a generally triangular path and while flanking the pins on the two canted wheels. Each belt is entrained in a groove in its associated wheel from a point prior to the impaling point and continues entrained until the above-mentioned second position, i.e., the point of tangency of the canted wheels with the supporting roll. Each belt is guided along a path on the supporting roll downstream of the point of tangency so as to effectively strip the stretched adhesive sheet from its impalement on the pins and press it firmly against the web traveling with the supporting roll. The belts are then entrained in the grooved idlers for a portion of the idler periphery so as to change direction and return to the grooves in the canted wheels. Advantageously, the belts are round belts, i.e., circular in cross-section. We have found this belt arrangement to be most effective in smartly stripping the stretched elastic webs from the pins while being relatively insensitive to adjustment.

The supporting roll lies in pressure contact with the belt means and the turn-around roll, and with them defines a compression area which extends around a substantial portion of its circumference. This compression area provides "marrying" pressure to adhere the elastic strip to the web. It provides this pressure for a predetermined time period - a period sufficiently long to ensure a permanent adhesive bond between the elastic sheet or strip and the web. The supporting roll continues to support the web with the sheet of elastic material on it until the web unites with other diaper components.

As the canted wheels deposit the elastic strip on the web at the second location and as the supporting roll rotates, the compression area receives the elastic strip in a stretched condition, the web, and the adhesive disposed between the strip and the web. The supporting roll rotates and moves these materials through the compression area. Thus, the belt assembly maintains pressure on the elastic material for a predetermined duration to adhere it to the web.

By use of the present invention there may be provided simple, high-speed, rotary operation which minimizes the cost of operation and, accordingly, the cost of the resulting product.

For a more complete understanding of this invention, one should now refer to the embodiment illustrated in greater detail in the accompanying darawings and described below by way of an example of the invention. In the drawings:

Fig.1 is a side elevational view (essentially schematic) of the apparatus of the present invention;

FIG. 2 is a fragmentary end elevational view taken along line 2-2 in FIG. 1;

FIG. 3 is a sectional view taken along line 3-3 in FIG. 2;

FIG. 4 is a sectional view taken along line 4-4 in FIG. 3;

FIG. 5 is a fragmentary schematic side elevational view showing the application of the elastic-equipped non-woven web to a moisture-impervious polyethylene web; and

FIG. 6 is a fragmentary perspective view of apparatus embodying the instant invention and is similar to the showing in FIG. 2.

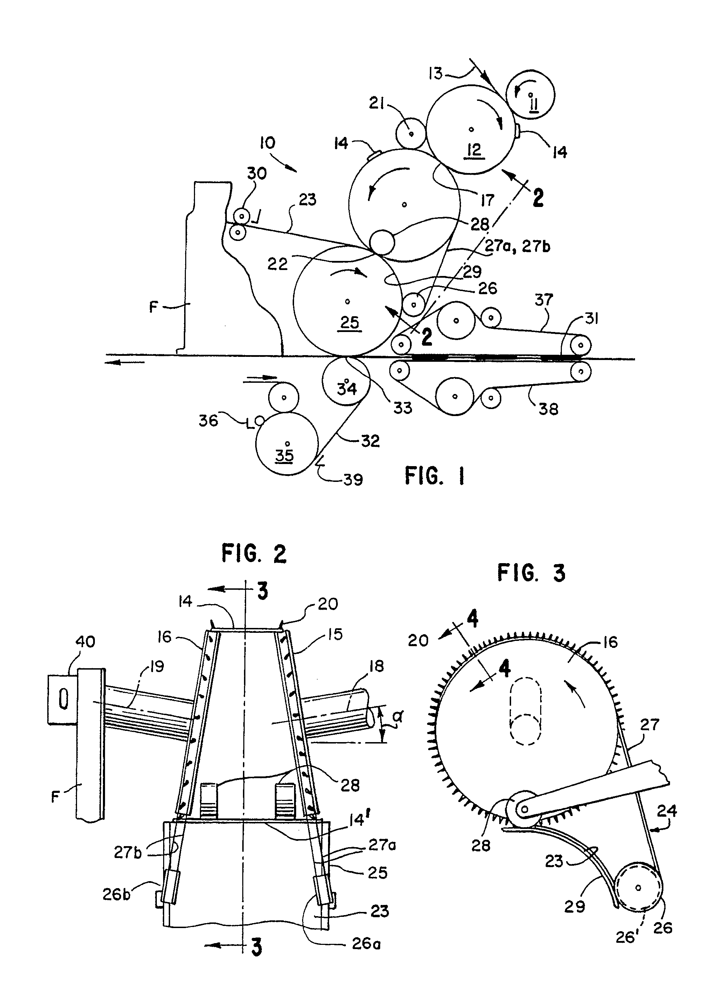

Turning to the drawings and referring first to FIG. 1, the apparatus of the present invention shown at 10 includes a frame F with a pair of side frames. This pair of side frames, united by suitable cross-members, defines the path of operation of the apparatus 10. FIG. 2 shows one of the side frames F'.

Still referring to FIG. 1, the frame F rotatably supports a cut-off roll 11 which cooperates with a vacuum anvil roll 12 to receive a continuous web of elastic sheet material 13 (e.g., polyurethane foam) which a supply roll (not shown) provides and to cut a strip 14 of this elastic material. The vacuum anvil roll 12 holds the strip 14 for a portion of its rotation and transfers it to a pair of canted wheels 15 and 16 (See FIG. 2) at 17.

The wheels 15 and 16 lie mounted on the frame F in transversely spaced apart relation for rotation about angularly related axes as at 18 relative to the wheel 15 and as at 19 relative to the wheel 16. The wheels 15 and 16 have the same diameter and include generally radially extending pins 20 mounted in generally equally circumferentially spaced relation as shown in FIG. 3.

As stated above, the vacuum anvil roll 12 holds the strip 14 for a portion of its rotation until the strip intersects, at 17, the envelope of rotation of the pins 20 on the wheels 15 and 16. Here, the pins 20 impale the elastic strip 14 at opposite ends of the strip, and the anvil roll releases the strip to transfer it to the wheel 15 and 16. (The strip extends between the wheels, releasably secured at one end to the wheel 15 and at its opposite end to the wheel 16.) After this transfer, a staking roll 21 presses the strip 14 into the pins 20 to assure that the elastic strip does not fall off of the pins.

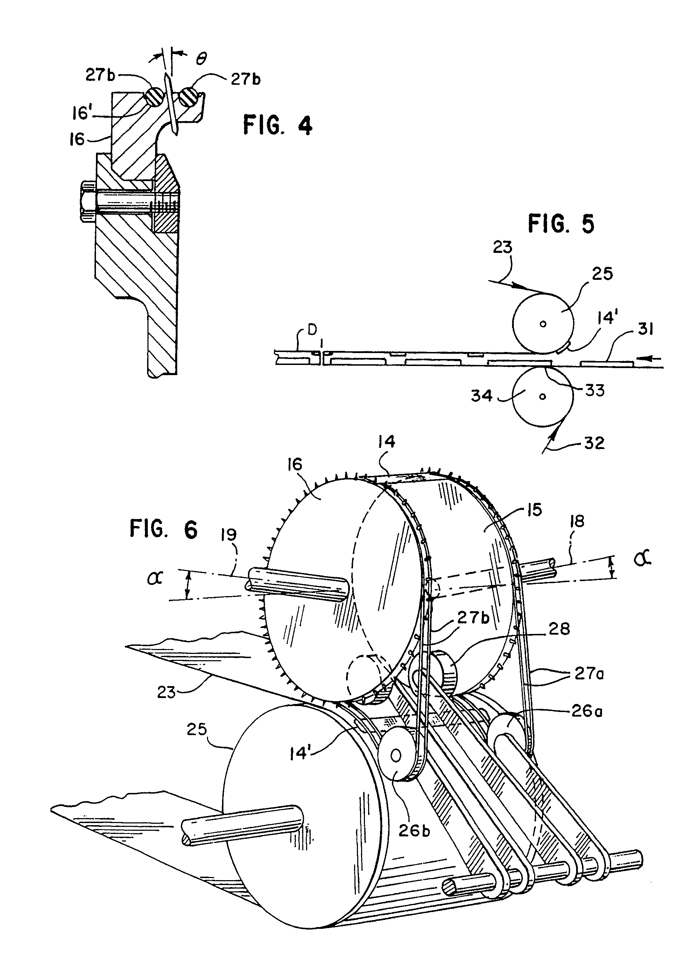

As the wheels 15 and 16 rotate (counterclockwise in FIG. 1) from the location 17 to a location 22, their peripheries move farther apart, stretching the strip 14 to a configuration designated 14' (See FIG. 2). At location 22, the wheels 15 and 16 place the strip 14' into alignment with the path of travel of a non-woven web 23. A roll and belt assembly generally designated 24 transfers the strip 14' to the non-woven web 23 as the web 23 travels on the supporting roll 25. The assembly 24 transfers the strip 14' transversely of the web 23 which a parent roll (not shown) provides. To facilitate this transfer, the pins 20 may lie slightly inclined as shown in FIG. 4. FIG. 4 shows the pins 20 inclined slightly outwardly, but they may also lie inclined slightly forwardly, depending upon the character of the elastic material and the physical arrangement of the apparatus 10.

The belt assembly 24 includes a pair of turnaround idlers 26a, 26b and two pairs of endless belts 27a and 27b which cooperate with portions of the peripheries of the canted wheels 15, 16 and the supporting roll 25. More particularly, the belts 27a, 27b ride in grooves as at 16' in the wheels 15, 16 (see FIG. 4), and in grooves in the idler sheaves as at 26' (see FIG. 3). Cooperating with the belt assembly is a pressure roll 28 at the point of tangency 22 (compare FIGS. 1 and 3).

The axis of rotation of the idler 26a lies parallel to the axis 18, and the axis of rotation of the idler 26b lies parallel to the axis 19. Alternatively, the axis of rotation of the idler 26a may lie slightly out of parallel alignment with the axis 18 due to independent installation of the idlers and the canted wheels. Similarly, the axis of rotation of the idler 26b may lie slightly out of parallel alignment with the axis 19 for the same reason.

The assembly 24 cooperates with the supporting roll 25 to define a compression area 29 which extends around a substantial portion of the circumference of the roll 25 -- see FIG. 3. (The roll 25 is a drum made of steel or any other suitable material.) Along the compression area 29, the assembly 24 and the supporting roll 25 provide "marrying" pressure to adhere the elastic strip 14' to the web 23. They provide this pressure for a predetermined time period -- a period sufficiently long to insure a permanent adhesive bond between the elastic and the web.

At 22, the strip 14', the web 23, and adhesive disposed between the strip and the web enter the compression area 29. An adhesive applying unit designated 30 (in FIG. 1) applies longitudinally spaced apart bands or areas of adhesive (e.g., a pressure sensitive adhesive) to web 23 before the web 23 comes into contact with the strip 14'. As the supporting roll 25 rotates, the web 23, adhesive, and strip 14' move through the compression area 29. The strip 14' moves farther away from the wheels 15, 16 and pulls away from the pins 20 which releasably secure it to the wheels. The belt and roll assembly 24 maintains pressure on the strip 14' for a predetermined duration to adhere it to the web 23. In a specific example, an apparatus 10 includes a supporting roll 25 with a 13 inch diameter and the compression area 29 has a path length of approximately 6.8 inches.

After leaving the compression area 29, the web 23 with the stretched strip 14' fixedly secured to it unites with spaced apart batts 31 and a moisture-impervious web 32 (e.g., polyurethane) at a nip 33 defined by the supporting roll 25 and a roll 34 which cooperates with it. The supporting roll 25 supports the web 23 at the compression area 29, and continues to support it at the nip 33 as well as between the nip and the compression area. A chill roll 35 advances the web 32 to the nip 33; and an adhesive applying unit 36 applies longitudinally spaced apart areas of adhesive to the web 32 at the roll 35. These areas of adhesive register with the strips 14' and secure them to the web 32 at the nip 33.

Conveyor belts as at 37 and 38 advance the batts 31 to the nip 33; and a fine line glue assembly 39 applies longitudinally spaced apart glue lines to the web 32. These glue lines register with the batts 31 and secure the batts 31 and the web 32 together at the nip 33. As shown in FIG. 5, the apparatus 10 cuts and severs the now combined webs 23 and 32 to form a series of discrete diapers as at D.

In the illustrated embodiment, the sheet 13 advances at a fraction of the speed of advance of the web 23 so that the strips 14 lie spaced apart on the canted wheels 15 and 16, a distance sufficient to install one strip per diaper. Each strip 14' spans the leading edge of one diaper and the trailing edge of the preceding diaper. The transverse severing which forms discrete diapers occurs midway of the strip 14'.

In operation, a continuous web of elastic sheet material 13 advances from a parent roll (not shown) towards a cut-off roll 11. The cut-off roll 11 cooperates with a vacuum anvil roll 12 to provide a series of discrete, spaced-apart segments or strips of elastic material 14. The vacuum anvil roll 12 moves the strips 14 into the pins 20 which receive the strips 14; and the staking roll 21 secures the strips 14 in place on the pins.

The wheels 15 and 16 move the strips 14 between the locations 17 and 22. They rotate counterclockwise and stretch the strips, increasing their length. At location 22, the strips 14' register with adhesive applied to the web 23; and the strips, the adhesive and the web 23 move through the compression area 29 as the strips pull away from the pins which releasably secure them to the wheels. The pressure provided in the compression area fixedly secures the strips 14' to the web 23. The web 23 with the strips 14' fixedly secured to it moves to the nip 33 defined by the rolls 25 and 34 where the web 23, the web 32, and the batts 31 unite.

While the above description and the drawings disclose one embodiment, one should understand, of course, that the invention is not limited to this embodiment. Those skilled in the art to which the invention pertains may make modifications and other embodiments employing the principles of this invention, particularly upon considering the foregoing teachings.

For example, one may phase the wheels 15 and 16 with a drive 40 (see FIG. 2). In phasing the wheels 15 and 16, the axes 18 and 19 are angularly related in the vertical plane (the angle a between a horizontal plane and the axes 18 and 19 in FIG. 2) as well as in a horizontal plane (the angle between the vertical plane and the axes 18 or 19) so that the wheels may provide different amounts of stretch with lesser or greater angular travel of the strip 14.

In addition, in some instances the apparatus 10 may include means for perforating the web 13 upstream rather than using a vacuum anvil roll and a cut-off roll. In such an alternative, the apparatus applies the web 13 directly to the pins 20 on the wheels 15 and 16. The perforation bonds break at the time of transfer to the web 23 under the influence of the assembly 24. However, in such an instance, the wheels operate at a surface speed slower than the speed of advance of the web 23 since the apparatus applies an elastic strip at predetermined intervals.

Therefore, by the appended claims, the applicants intend to cover any modifications and other embodiments which incorporate those features which constitute the essential features of this invention.

## Claims

1. An apparatus for making disposable diapers having a stretchable waistband, said apparatus comprising:

   a frame (F), means on said frame providing a path of travel for a diaper component web, a pair of spaced-apart, substantially identical wheels (15,16) mounted on said frame for rotation about angularly related axes whereby corresponding points on the peripheries of said wheels (15,16) are closer together at a first position than at a second position, said second position being spaced circumferentially from said first position, pin means (20) disposed on the periphery of each of said wheels (15,16) for receiving elastic sheet material (14) and releasably securing it to said wheels (15,16), first transfer means (12,14) disposed on said frame for transferring sheet material to said pin means (20) at said first position, said sheet material (14) spanning the space between said wheels (15,16), and second transfer means disposed on said frame (F) in said path for removing said elastic material (14) from said pin means (20) at said second position and transferring said elastic material to a diaper component web (23), said second transfer means including a supporting roll (25) rotatably mounted on said frame (F) adjacent said pair of wheels (15,16) and a belt assembly (24), said assembly including a turn-around means (26a,26b) mounted on said frame (F) downstream in said path from said second position and endless belt means (27a,27b) entrained on said turn-around means (26a,26b), said pair of wheels (15,16) and said supporting roll (25), said belt means (27a,27b) underlying said sheet material on said wheels (15,16) and overlying said sheet material and web on said supporting roll (25).

2. An apparatus as claimed in claim 1 wherein said wheels (15, 16) are equipped with belt entraining grooves (16') adjacent said pin means (20) and said turn-around means (26a, 26b) is equipped with belt entraining grooves (26').

3. An apparatus as claimed in claim 1 or 2, in which said belts are circular in cross-section.

4. A method of manufacturing disposable diapers using a pair of canted wheels (15, 16) rotated about angularly related axes, said wheels (15, 16) including circumferentially spaced-apart pin means (20) for impaling receiving elastic sheet material (14) and releasably securing said sheet material (14) to said wheels (15, 16), said method comprising the steps of:

   (a) advancing an elongated diaper component web (23) along a predetermined path,
   (b) rotatably advancing the elastic sheet material (14) with said canted wheels (15, 16) and simultaneously stretching said elastic sheet material (14),
   (c) stripping said stretched elastic material from said pin means (20) by applying a localized force to said material immediately adjacent said pin means (20) and to apply said material to said web (23), and
   (d) continuing to apply said localized force to said material and web in said path.

FIG. 1

FIG. 2

FIG. 3

FIG. 4

FIG. 5

FIG. 6

**European Patent Office**

**EUROPEAN SEARCH REPORT**

Application Number

**EP 90 31 2451**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 338 662 (PAPER CONVERTING)<br>* Abstract; figure *<br><br>— — — — — | 1 | A 61 F 13/15 |

|  |  |  | TECHNICAL FIELDS SEARCHED (Int. Cl.5) |
|---|---|---|---|
|  |  |  | A 61 F |

The present search report has been drawn up for all claims

| Place of search | Date of completion of search | Examiner |
|---|---|---|
| The Hague | 27 May 91 | PEETERS S. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document